# EUROPEAN PATENT APPLICATION

(11) **EP 4 166 932 A1**
(43) Date of publication of application: **19.04.2023**
(21) Application number: 21202600.9
(22) Date of filing: 14.10.2021
(51) Int. Cl.: G01N 21/78, G01S 17/88

(54) **ENHANCED METHOD FOR THE DETERMINATION OF AN ANALYTE CONCENTRATION IN BODILY FLUID**

(71) Applicant: F. Hoffmann-La Roche AG, 4070 Basel (CH); Roche Diabetes Care GmbH, 68305 Mannheim (DE)
(72) Inventor: ALPEROWITZ, Lukas, 70619 Stuttgart (DE); BERG, Max, 68305 Mannheim (DE); BODLÉE, Florian, 70619 Stuttgart (DE); HAILER, Fredrik, 68305 Mannheim (DE); LIMBURG, Bernd, 68305 Mannheim (DE)
(74) Representative: Riwotzki, Karsten

(57) **Abstract**

The present invention relates to an analytical method for determining a concentration of an analyte in a bodily fluid by using a mobile device having at least one camera, at least one lidar sensor, at least one processor, and at least one display, wherein the at least one camera and the at least one lidar sensor comprise an at least partially overlapping field of view, the method comprising the following steps:
a) providing at least one object, the at least one object being selected from the list comprising: an optical test element having a reagent test region, a color reference card having a reagent test region, a color reference card adapted to be associated with an optical test element having a reagent test region; wherein the reagent test region is adapted for application of a sample of the bodily fluid, and wherein the reagent test region is adapted to undergo, at least partially, a color formation reaction when the sample of the bodily fluid is applied to the reagent test region;
b1) prompting, by the display, a user to apply a drop of the bodily fluid to the reagent test region and/or prompting, by the display, a user to confirm application of a drop of the bodily fluid to the reagent test region;
b2) prompting, by the display, the user to provide the at least one object within the at least partially overlapping field of view of the at least one camera and the at least one lidar sensor;
c) generating, by the processor, a lidar measurement data set at least for the object by receiving output data from the at least one lidar sensor, the lidar measurement data set representing a three-dimensional structure of at least a part of the object;
d) comparing, by the processor, the lidar measurement data set from step c) to a pre-generated lidar data set for the object, the pre-generated lidar data set representing a three-dimensional structure of the entire object, thereby obtaining an item of information on a degree of congruence of the lidar measurement data set and the pre-generated lidar data set; and
e1) if the item of information from step d) indicates a degree of congruence equal to or above a pre-determined minimum degree of congruence: capturing, by the at least one camera, a measurement image of at least a part of the reagent test region having the sample of the bodily fluid applied thereto, and determining the concentration of the analyte in the bodily fluid based at least on the measurement image captured; or
e2) if the item of information from step d) indicates a degree of congruence below a pre-determined minimum degree of congruence:
- at least temporarily not allowing the capturing, by the at least one camera, of a measurement image of at least a part of the reagent test region having the sample of the bodily fluid applied thereto; and/or
- indicating, by the display, a warning to the user; and/or
- indicating, by the display, instructions to the user to take some appropriate action in order to capture, by the at least one camera, a measurement image of at least a part of the reagent test region having the sample of the bodily fluid applied thereto.

## Description

### Technical Field

The present invention relates to a method of determining a concentration of an analyte in a bodily fluid, using at least one mobile device having a camera and a processor. Further, the invention relates to a mobile device having a camera and a processor for carrying out the method, to a kit comprising a mobile device having a camera and a processor, to computer programs and computer-readable storage media. The methods, mobile devices, computer programs and storage media specifically may be used in medical diagnostics, for example in order to qualitatively or quantitatively detect one or more analytes in body fluids, such as for detecting glucose in blood or interstitial fluid.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary.

Generally, devices and methods known to the skilled person make use of test elements comprising one or more test chemicals, which, in the presence of the analyte to be detected, are capable of performing one or more detectable detection reactions, such as optically detectable detection reactions. With regard to the test chemicals comprised in test elements, reference may be made e.g. to J. Hoenes et al.: The Technology Behind Glucose Meters: Test Strips, Diabetes Technology & Therapeutics, Volume 10, Supplement 1, 2008, S-10 to S-26.

Specifically, in analytical measurements based on color formation reactions, the color change which is due to the detection reaction is evaluated. Besides using dedicated analytical devices, such as handheld blood glucose meters, the use of generally available electronics such as smart phones and portable computers or other mobile devices has become more and more popular over the recent years. For example, methods and devices for optically measuring analytes by using a mobile device having a camera, addressing various specific aspects to be considered in such measurement scenarios, are described in EP3527972A1, WO 2019/238500A1, EP3650843A1, and EP3651162A1. In order to enhance such measurement procedures using mobile devices, it has also been suggested to take into account additional data which is available from sensors present in a mobile device, such as from accelerators, gyroscopes, and the like. Such data may e.g. be used for improved user guidance, as described in international patent application PCT/EP2021/068431.

Apart from sensors which are currently widely used in mobile devices, such as accelerators or gyroscopes, it may be expected that more and more mobile devices will be equipped with further additional sensors which are currently available only in a very small number of mobile devices. One such example are lidar sensors, the term lidar (or ladar) generally being known as "light detection and ranging" or "laser imaging, detection, and ranging", a method for determining ranges (variable distances) by using reflected laser light. With lidar, it is possible to make digital 3-D representations of a surrounding or of objects. Lidar has various terrestrial, airborne, and mobile applications, and in particular is commonly used to make high-resolution maps, with applications e.g. in geography, geomorphology, atmospheric physics, and many more.

For example, lidar is also used in navigation or control systems of autonomous vehicles for detection of obstacles. In this context, a combined use of 3-D lidar and a color camera has been described for multiple object detection, and also for the tracking of moving objects. (Hwang, Soonmin et al. (2016) "Fast Multiple Objects Detection and Tracking Fusing Color Camera and 3D LIDAR for Intelligent Vehicles", 13th Int. Conf. on Ubiquitous Robots and Ambient Intelligence (URAI), ISBN 978-1-5090-0821-6). Both inputs from lidar and a camera are obtained in parallel, and the color image from the camera is calibrated with the lidar. In a segmentation step, using statistical analysis, the 3-D points are divided into several groups, based on the distance from the sensor. This way, foreground objects can be separated from background objects, wherein object proposals in the 2-D image may be used. Detection, on the one hand, comprises object detection in the 2-D image, and on the other hand, comprises object detection in the 3-D space as detected by the lidar sensor, whereby local and global histograms may be extracted to represent a certain object. For the merging of the results from 2-D image and 3-D space object detection, Scores calibration can be performed, whereby a single confidence score from both detectors can be obtained in the form of probability. (Xu, Philippe et al. (2014) "Evidential combination of pedestrian detectors", Proceedings Brit. Machine Vision Conf., BMVA Press). For actual tracking (e.g. of a car in motion), additional steps are necessary, such as associating moving objects in subsequent frames over time.

Furthermore, as far as user guidance during measurements employing a mobile device is concerned, WO2021/105222A1 provides for some visual indication on the display of the mobile device, which may include the use of augmented reality, particularly for scenarios where a first and a second image (such as a blank or reference image, and a subsequent measurement image) should be captured essentially at the same position in order to minimize any impact of environmental factors such as ambient lighting conditions.

In order to properly take into account reference colors for the evaluation of color formation from a detection reaction, improvements result from using color reference charts along with analytical test strips. This approach is particularly beneficial if images are captured both of the color reference chart and the test strip simultaneously. Examples for such a procedure are described in international patent application no. PCT/EP2021/065087 by the applicant, while examples of suitable color reference cards are disclosed in EP application no. 20190454.7 by the applicant.

If a color reference card is used together with a test strip, various orientations of the mobile device relative to the color reference card may be allowed, enabled e.g. by specific markers on the upper surface of the card (such as ArUco codes). While user handling is improved thereby, relative measurements, such as capturing a first blank image and then a final measurement image, becomes more complex and difficult. For example, while a determination of the distance between a camera of a mobile device and the color reference card may be possible by taking into account different focal length, focal point, resolution, etc., such an approach may also be complicated and slow.

Moreover, a color reference card represents an object of certain dimensions, for example being larger than a human's finger. Hence, the risk of portions of such a color reference card being partially hidden by another object during a measurement, such as by one or more fingers of a user, is encreased. However, such coverage of parts of a color reference card by objects or obstacles may not be distinguishable from e.g. shadows or from some damage to the card. Therefore, in such cases, it will not be possible to provide appropriate feedback to a user.

Accordingly, despite the advantages involved in using mobile computing devices for the purpose of performing an analytical measurement, one of the remaining technical challenges still is to increase the usability, and thereby to also increase the reliability of such measurement scenarios.

### Problem to be solved

It is therefore desirable to provide devices and methods which at least partially address the above-mentioned challenge. Specifically, it is desirable to provide devices and methods which allow for a reliable mobile-based determination of a concentration of an analyte in a bodily fluid, whereby handling efficiency is increaased, in particular by appropriate user guidance.

### Summary

This problem is addressed by an analytical method for determining a concentration of an analyte in a bodily fluid by using a mobile device having at least one camera, at least one lidar sensor, at least one processor, and at least one display; further, by a mobile device having at least one camera, at least one lidar sensor, at least one processor, and at least one display, by a kit comprising a mobile device and an object suiable for an optical measurement, and by computer programs and computer-readable storage media, with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, a method for determining a concentration of an analyte in a bodily fluid is disclosed, the method comprising using a mobile device having a camera and a processor. The method comprises the following steps which, as an example, may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed. Generally, the method comprises capturing at least one image of at least a part of an optical test strip, or of a color refernce card, having a sample of the bodily fluid applied onto a reagent test region of the test strip or of the color refernce card, wherein the capturing comprises using the camera of the mobile device. The at least one image captured comprises at least a part of the reagent test region having the sample of the bodily fluid applied thereto. The method further comprises determining, by the processor, the analyte concentration from the image captured, based on a color formation reaction at the reagent test region having the sample of the bodily fluid applied thereto.

Without narrowing the scope, the invention specifically may be described with respect to blood glucose measurements. It shall be noted, however, that the present invention may also be used for other types of analytical measurements using test elements.

The term "determining a concentration of an analyte in a bodily fluid", also referred to as an "analytical measurement", as used herein, is a broad term and is to be given its ordinary and customary meaning to a person of ordinary skill in the art and is not to be limited to a special or customized meaning. The term specifically may refer, without limitation, to a quantitatively and/or qualitatively determination of at least one analyte in an arbitrary sample or aliquot of bodily fluid. For example, the bodily fluid may comprise one or more of blood, interstitial fluid, urine, saliva or other types of body fluids, particularly blood; specifically, the bodily fluid may comprise specific portions of blood like serum or plasma. The result of the determining of the concentration, as an example, may be a concentration of the analyte and/or the presence or absence of the analyte to be determined. Specifically, as an example, the analytical measurement may be a blood glucose measurement, thus the result of the analytical measurement may for example be a blood glucose concentration. In particular, an analytical measurement result value may be determined by the analytical measurement.

Consequently, the term "analyte concentration value", often also referred to as "analytical measurement result value", as used herein, is a broad term and is to be given its ordinary and customary meaning to a person of ordinary skill in the art and is not to be limited to a special or customized meaning. The term specifically may refer, without limitation, to a numerical indication of an analyte concentration in a sample.

The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value.

The method, as outlined above, comprises using at least one mobile device having at least one camera. The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, the mobile device may also refer to a tablet computer or another type of portable computer having at least one camera and at least one processor.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The term "lidar" as used herein is a broad term and is to be given its ordinary and customary meaning to a person of ordinary skill in the art and is not to be limited to a special or customized meaning. Lidar (or "LIDAR", "LADAR", or "3-D laser scanning") generally is known as "light detection and ranging" or "laser imaging, detection, and ranging". It is a method for determining ranges (i.e. variable distances) by targeting an object with a laser, usually a pulsed laser, and measuring the time for the scattered or reflected light to return to the receiver. For making digital 3-D representations of a surrounding or of any objects, lidar usually relies on differences in laser return times, but may additionally rely on varying laser wavelengths. Conventional scanning lidar generally uses a collimated laser beam that illuminates a single point at a time, and the beam is raster scanned to illuminate the field of view, in a point-by-point manner. A time-of-flight camera, comprising a receiver, gathers information about both the 3-D location and intensity of the light incident on it, which information may be captured in one or more frames (herein also referred to as "lidar image", or "image" of a lidar sensor). To this end, it may be sufficient for the camera to contain a point sensor. In so-called flash lidar, wherein the entire field of view is illuminated in a single pulse by a diverging laser beam, the camera may contain a 1-D or a 2-D sensor array, each pixel of which collects 3-D location and intensity information. In both cases, lidar and flash lidar, the depth information is collected using the time of flight of the laser pulse (i.e., the time it takes each laser pulse to impinge on the target and return to the receiver), such that the pulsing of the laser and the acquisition by the receiver are required to be synchronized. As a result, "pictures of distance" (i.e. "lidar images") can be captured, as opposed to, or in addition to, conventional images made of colors. From the context of navigation or control systems of autonomous vehicles for detection of obstacles, a combined use of 3-D lidar and a color camera has been described for multiple object detection, as described herein above.

Herein, the terms "lidar" and "lidar sensor" are used as synonyms, and both terms specifically may refer, without limitation, to a system, a device, or one or more components thereof, having the capability of a time-of-flight camera which provides for recording or capturing spatially resolved three-dimensional data or information of one or more objects within the field of view of the time-of-flight camera. Specifically, the term "lidar" may relate to a scanning lidar system which emits a plurality of laser pulses at different parts of a scene during a fractional period of time, i.e. within a very short period of time. Thereby, real-time user guidance may be provided based on data received from the lidar sensor, such as user guidance for adjusting an orientation of the mobile device relative to an object, specifically relative to the at least one obj ect.

For example, a lidar sensor to be used according to the present invention may comprise at least one laser element for emission of a pulsed collimated laser beam, at least one means for raster scanning the beam to illuminate the field of view of the time-of-flight camera in a point-by-point manner (i.e. a single "lidar point" at a time), at least one receiver configured for recording or capturing the light scattered or reflected by one or more objects within the field of view of the time-of-flight camera, specifically including recording or capturing an intensity of the light scattered or reflected, and at least one timer configured for detecting or recording the time it takes for a pulse of the emitted laser beam to return to the receiver. The resolution of a lidar sensor to be used in the present invention may be selected such that a relatively high number of single "lidar points" will impinge upon the at least one object to be detected, e.g. a plurality of single "lidar points" of at least 20, at least 30, or at least 50. A lidar sensor which may be suitably used may have a resolution of 256 x 192 single "lidar points" which are raster scanned by the pulsed laser beam wherein specifically each pulse of the laser beam may raster scan one single "lidar point". Commercially available mobile devices equipped with suitable lidar sensors comprise, for example, tablet computers like the Apple^{®} iPad Pro 2020^{®}, and smartphones like the Apple^{®} iPhone 12 Pro^{®} and Pro Max^{®}. These mobile devices each comprise a lidar sensor which, by using lidar scanning, can perform time-of-flight calculations to generate a 3D map of the surrounding area.

The method further comprises using at least one of: an optical test element having a reagent test region, a color reference card having a reagent test region, and a color reference card adapted to be associated with an optical test element having a reagent test region. The reagent test region is adapted for application of a sample of the bodily fluid, and the reagent test region is adapted to undergo, at least partially, a color formation reaction when the sample of the bodily fluid is applied to the reagent test region. The reagent test region may also be referred to as a "test field" herein. The term "optical test element" as used herein is a broad term and is to be given its ordinary and customary meaning to a person of ordinary skill in the art and is not to be limited to a special or customized meaning. The term specifically may refer, without limitation, to an arbitrary element or device configured for performing a color-change detection reaction. The optical test element may also be referred to as test strip or test element, wherein all three terms may refer to the same element. The optical test element and/or the color reference card may particularly have a reagent test region containing at least one test chemical for detecting at least one analyte. The optical test element, as an example, may comprise at least one substrate, such as at least one carrier, with the at least one reagent test region applied thereto or integrated therein. In particular, the optical test element may further comprise one or more reference areas, such as a white field and/or a black field. Additionally or alternatively, the substrate or carrier itself may be or may comprise such a reference area. As an example, the at least one carrier may be strip-shaped, thereby rendering the test element a test strip. These test strips are generally widely in use and available. One test strip may carry a single test field or a plurality of test fields having identical or different test chemicals comprised therein. The color reference card may comprise analogous features as described herein above for the optical test strip. Particularly, the color reference card may be provided in credit card format, i.e. in the size and form of a conventional credit card made of plastic. Usually, such a card-sized color reference card exhibits a plurality of reference areas, such as a white field, a black field and/or grey fields. Additionally or alternatively, a color reference card may exhibit a plurality of reference areas having a variety of reference colors, said reference colors having colors other than white, black or grey.

As further used herein, the term "reagent test region" (also referred to as a "test field" herein) is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the test field having the test chemical comprised therein. With regard to the test chemicals comprised in optical test strips, as an example reference is made to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

As outlined above, the method generally comprises capturing at least one image of at least a part of the reagent test region having the sample of the bodily fluid applied thereto, by using the camera. The term "capturing at least one image" as used herein is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by the user action or may automatically be initiated, e.g. once the presence of the at least one object within a field of view and/or within a predetermined sector of the field of view of the camera is automatically detected. These automatic image acquisition techniques are known e.g. in the field of automatic barcode readers, such as from automatic barcode reading apps. The capturing of the images may take place, as an example, by acquiring a stream or "live stream" of images with the camera, wherein one or more of the images, automatically or by user interaction such as pushing a button, are stored and used as at least one first image or as at least one second image, respectively. The image acquisition may be supported by a processor of the mobile device, and the storing of the images may take place in a data storage device of the mobile device.

The capturing of the at least one image may comprise capturing at least one image with having the sample of the bodily fluid applied to the test strip and, further and optionally, such as before capturing the image with the sample applied to the test strip, capturing at least one image without having the sample of the body fluid applied to the test strip. The latter image specifically may be used for comparative purposes and may also be referred to as a "blank image" or "dry image". The sample application generally may take place, as an example, directly or indirectly, e.g. via at least one capillary element. The at least one image captured after sample application may typically also be referred to as the "wet image", even though the sample may have dried when the image is actually captured. The wet image typically may be taken after having waited for at least a predetermined waiting time, such as after five seconds or more, in order to allow for the detection reaction to take place. Thus, as an example, the method may comprise, between taking an optional dry image and at least one wet image, waiting for at least a predetermined minimum amount of time. This predetermined minimum amount of time specifically may be sufficient for a detection reaction to take place in the test strip. As an example, the minimum amount of waiting time may be at least 5 s.

The method comprises determining the analyte concentration, particularly an analyte concentration value, from color formation of the test field. Thus, the method may be an analytical measurement including a change of at least one optical property of an optical test field, which change may be measured or determined visually by using the camera. Specifically, the analytical measurement may be or may comprise a color formation reaction in the presence of the at least one analyte to be determined. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction. The color formation may be detected by the mobile device, such as by a processor of the mobile device, and may be evaluated quantitatively, such as by deriving, from the at least one image, at least one parameter quantifying or characterizing the color formation of the test field due to the presence of the analyte in the bodily fluid. To this end, one or more specific color coordinates may be used. Thus, the mobile device and specifically the processor of the mobile device may be configured for determining a color change by determining a change of one or more color coordinates taking place due to the detection reaction.

The at least one analyte concentration, particularly analyte concentration value, is determined from the color formation of the test field. For this purpose, at least one image is used. The analyte concentration value, as an example, may be a numerical value indicator of a result of the analytical measurement, such as indicative of the concentration of at least one analyte in the sample, such as a blood glucose concentration.

The method may further comprise the step of displaying the analyte concentration value, such as on a display of the mobile device. Additionally or alternatively, the method may comprise storing the at least one analyte concentration value in at least one data storage device of the mobile device. Again additionally or alternatively, the method may further comprise transmitting the at least one analyte concentration value via at least one interface and/or via at least one data transmission network, such as to another computer, e.g. for further evaluation.

Accordingly, in the first aspect, the present invention particularly relates to an analytical method for determining a concentration of an analyte in a bodily fluid by using a mobile device having at least one camera, at least one lidar sensor, at least one processor, and at least one display, wherein the at least one camera and the at least one lidar sensor comprise an at least partially overlapping field of view, the method comprising the following steps:
a) providing at least one object, the at least one object being selected from the list comprising: an optical test element having a reagent test region, a color reference card having a reagent test region, a color reference card adapted to be associated with an optical test element having a reagent test region; wherein the reagent test region is adapted for application of a sample of the bodily fluid, and wherein the reagent test region is adapted to undergo, at least partially, a color formation reaction when the sample of the bodily fluid is applied to the reagent test region;
b1) prompting, by the display, a user to apply a drop of the bodily fluid to the reagent test region and/or prompting, by the display, a user to confirm application of a drop of the bodily fluid to the reagent test region;
b2) prompting, by the display, the user to provide the at least one object within the at least partially overlapping field of view of the at least one camera and the at least one lidar sensor;
c) generating, by the processor, a lidar measurement data set at least for the object by receiving output data from the at least one lidar sensor, the lidar measurement data set representing a three-dimensional structure of at least a part of the object;
d) comparing, by the processor, the lidar measurement data set from step c) to a pre-generated lidar data set for the object, the pre-generated lidar data set representing a three-dimensional structure of the entire object, thereby obtaining an item of information on a degree of congruence of the lidar measurement data set and the pre-generated lidar data set; and
e1) if the item of information from step d) indicates a degree of congruence equal to or above a pre-determined minimum degree of congruence: capturing, by the at least one camera, a measurement image of at least a part of the reagent test region having the sample of the bodily fluid applied thereto, and determining the concentration of the analyte in the bodily fluid based at least on the measurement image captured; or
e2) if the item of information from step d) indicates a degree of congruence below a pre-determined minimum degree of congruence:
   - at least temporarily not allowing the capturing, by the at least one camera, of a measurement image of at least a part of the reagent test region having the sample of the bodily fluid applied thereto; and/or
   - indicating, by the display, a warning to the user; and/or
   - indicating, by the display, instructions to the user to take some appropriate action in order to capture, by the at least one camera, a measurement image of at least a part of the reagent test region having the sample of the bodily fluid applied thereto.

The method proposed provides for a reliable mobile-based determination of a concentration of an analyte in a bodily fluid, particularly by reducing the occurrence of errors from inappropriate user handling during a measurement procedure. Thereby, the handling efficiency of mobile-based determination of a concentration of an analyte in a bodily fluid is increaased, specifically by providing enhanced feedback to the user, such as more appropriate user guidance.

The mobile device has at least one camera, e.g. one, two or three cameras. The cameras may be located on the rear side of the mobile device, on the front side of the mobile device, or on both sides, wherein the front side is the side having the display and the rear side is the side opposite the side having the display. Specifically, at least one of the cameras is located on the rear side of the mobile device. The mobile device has at least one lidar sensor, e.g. one or two, wherein the at least one lidar sensor is located on the same side of the mobile device which has the at least one camera. Specifically, the at least one lidar sensor is located on the rear side of the mobile device. The at least one camera and the at least one lidar sensor may be located in close proximity to each other. Furthermore, the at least one camera and the at least one lidar sensor comprise an at least partially overlapping field of view. For example, the overlap of the field of view may be at least 50%, specifically at least 75%, and more specifically at least 90%. Advantageously, the field of view of the at least one camera and of the at least one lidar sensor may overlap essentially completely.

In step a), at least one object is provided, wherein the at least one object is selected from the list comprising: an optical test element having a reagent test region, a color reference card having a reagent test region, a color reference card adapted to be associated with an optical test element having a reagent test region. The terms "optical test element", "color reference card" and "reagent test region" may specifically refer to the definitions for each of the corresponding terms given herein above. The reagent test region is adapted for application of a sample of the bodily fluid, such as blood, and the reagent test region is adapted to undergo, fully or at least partially, a color formation reaction when the sample of the bodily fluid is applied to the reagent test region. A color reference card which is "adapted to be associated with" an optical test element having a reagent test region may refer to a color reference card which can be removably or fixedly connected to a test element, such as to a test strip, having a reagent test region. In particular, a test element or test strip may be attached to such a color reference card by some fixation means, like a slot or guide rails or something similar, for insertion of the test strip, such that the test element remains in a fixed position relative to the color reference card. Examples of appropriate color reference cards of this type are disclosed e.g. in EP application no. 20190454.7 by the applicant. Alternatively, a color reference card may be "adapted to be associated with" an optical test element having a reagent test region in such a manner that the color reference card is used together with the optical test element without fixation elements to hold the test element in place. In particular, an optical test element may simply be placed next to, or on top of, the color reference card, e.g. at a predetermined edge of the color reference card, or at a predetermined location on the top surface of the color reference card. This way, both the lidar sensor and the camera of the mobile device may, in any subsequent steps of the method, receive input data including both the color reference card and the test element.

In step b1), a user is prompted, by the display, to apply a drop of the bodily fluid to the reagent test region. Additionally or alternatively, in step b1), a user is prompted, by the display, to confirm application of a drop of the bodily fluid to the reagent test region. In step b2), the user is prompted, by the display, to provide the at least one object within the at least partially overlapping field of view of the at least one camera and the at least one lidar sensor. The prompting may, in each case, be performed by a corresponding message, icon or other graphical representation on the display. Moreover, other means for prompting the user may comprise acoustic and/or haptic signals, such as tone signals, alarms, vibration, etc. Particularly, steps b1) and b2) may be carried out simultaneously or in the reverse order, e.g. firstly step b2) and subsequently step b1).

In step c), a lidar measurement data set is generated, by the processor, at least for the at least one object by receiving output data from the at least one lidar sensor, wherein the lidar measurement data set generated represents a three-dimensional structure of at least a part of the object or of the complete object. Particularly, if any obstacle, such as a user's finger, obstructs detection of the complete object by the lidar sensor, then the lidar measurement data set can only be generated so as to represent a three-dimensional structure of only a part of the object, namely the part of the object which is within the field of view of the lidar sensor and which is not obstructed by any obstacle.

In step d), the lidar measurement data set from step c) is compared, by the processor, to a pre-generated lidar data set for the at least one object. The pre-generated lidar data set represents a three-dimensional structure of the entire object. Usually, the pre-generated lidar data set is generated in a training procedure, wherein said training procedure may involve the use of an artificial neural network. Specifically, the pre-generated lidar data set may be generated by using the same type of lidar sensor which is used in the mobile device as employed in the method of the present invention. However, other lidar sensors, including different hardware and/or software components, may be used as well. Furthermore, the pre-generated lidar data set generally is generated by using the same type of object, or of objects, which is used in the present method for determining a concentration of an analyte in a bodily fluid. Accordingly, the object used in a training procedure for generating the pre-generated lidar data set may specifically be selected from the list comprising: an optical test element having a reagent test region, a color reference card having a reagent test region, a color reference card adapted to be associated with an optical test element having a reagent test region. As a result of such a training procedure, a pre-generated lidar data set for the at least one object is obtained, wherein the pre-generated lidar data set represents a three-dimensional structure of the entire object.

From the comparison in step d), an item of information on a degree of congruence of the lidar measurement data set and the pre-generated lidar data set is obtained. The degree of congruence thus represents an extent of overlap, qualitatively or quantitatively, of the lidar measurement data set on the one hand and the pre-generated lidar data set on the other hand. Thereby, the processor may determine if there is a low, medium or high extent of overlap of both lidar data sets. Alternatively, the processor may simply distinguish a low from a high extent of overlap of both lidar data sets. Additionally or alternatively, the processor may determine a percentage of overlap of both lidar data sets. The skilled person will appreciate that the determination of the extent of overlap depends on wether the at least one object is fully detectable ("visible") within the field of view of the lidar sensor, or if it is only partially detectable ("visible") by the lidar sensor. For example, the object may only be partially detectable if an obstacle, such as a user's finger, obstructs detection of the complete object by the lidar sensor.

The degree of congruence of the lidar measurement data set and the pre-generated lidar data set, as indicated by the item of information from step d), is compared to a minimum degree of congruence. Herein, the minimum degree of congruence is selected such that the at least one object is positively identified within the lidar measurement data set. Moreover, if the at least one object is only partially detectable by the lidar sensor, the minimum degree of congruence is further selected such that the extent of partial overlap of the lidar measurement data set and the pre-generated lidar data set is sufficient to ensure a reliable analyte measurement according to step e1). In particular, for a reliable analyte measurement, the extent of partial overlap of the two lidar data sets includes, in the overlapping portions of the two lidar data sets, at least the reagent test field of the optical test element, and advantageously additional parts of the optical test element, such as reference fields, e.g. black, white grey and/or colored reference fields.

Accordingly, if the item of information from step d) indicates a degree of congruence equal to or above a pre-determined minimum degree of congruence, the method further comprises step e1), i.e. capturing, by the at least one camera, a measurement image of the reagent test region, or of at least a part thereof, having the sample of the bodily fluid applied thereto. Then, the concentration of the analyte in the bodily fluid is determined, by the processor, based at least on the measurement image captured.

Alternatively, if the item of information from step d) indicates a degree of congruence below a pre-determined minimum degree of congruence, the method further comprises step e2), i.e. carrying out at least one of the following:
- at least temporarily not allowing the capturing, by the at least one camera, of a measurement image of at least a part of the reagent test region having the sample of the bodily fluid applied thereto;
- indicating, by the display, a warning to the user; and
- indicating, by the display, instructions to the user to take some appropriate action in order to capture, by the at least one camera, a measurement image of at least a part of the reagent test region having the sample of the bodily fluid applied thereto.

A warning and/or instructions may be indicated to the user according to step e2) by a corresponding message, icon or other graphical representation on the display. Moreover, other means for indicating a warning and/or instructions to the user may comprise acoustic and/or haptic signals, such as tone signals, alarms, vibration, etc.

The appropriate action to be taken by the user in step e2) may be at least one of: clearing the overlapping field of view, at least in between the camera and the at least one object, of any obstacles; moving the mobile device in at least one direction, relative to the at least one object; and changing an angle of orientation of the mobile device relative to the at least one object; such that the overlapping field of view, at least in between the camera and the at least one object, is clear of any obstacles, such as e.g. a finger of a human user.

The pre-determined minimum degree of congruence in steps e1) and e2) represents a threshold. Only if the item of information from step d) indicates a degree of congruence equal to or above said threshold, i.e. equal to or above said pre-determined minimum degree of congruence, the method will further proceed to step e1) which includes actually determining the analyte concentration.

Specifically, if the item of information from step d) is selected from a group consisting of a low degree of congruence and a high degree of congruence, the pre-determined minimum degree of congruence may be set as high. In other words, in this case the processor determines the degree of congruence of the lidar measurement data set and the pre-generated lidar data set on a binary scale, resulting either in a low degree of congruence or in a high degree of congruence; and thus either the low degree of congruence or the high degree of congruence, as the case may be for any concrete performance of the method of the present invention, is indicated by the item of information from step d). Since in this case the pre-determined minimum degree of congruence is set as high, as mentioned before, a determination of the analyte concentration in step e1) can only be carried out if the item of information from step d) indicates a high degree of congruence (i.e. a degree of congruence equal to or above the applicable threshold).

Alternatively, if the item of information from step d) is selected from a group consisting of a low degree of congruence, a medium degree of congruence, and a high degree of congruence, the pre-determined minimum degree of congruence may be set as medium or high, and specifically as high. Thus, in this case, a determination of the analyte concentration in step e1) can only be carried out if the item of information from step d) indicates at least a medium, i.e. a medium or a high, degree of congruence (i.e. a degree of congruence equal to or above the applicable threshold). Or, in case the pre-determined minimum degree of congruence is specifically set as high, a determination of the analyte concentration in step e1) can only be carried out if the item of information from step d) indicates a high degree of congruence.

Still alternatively, if the item of information from step d) is selected to represent the degree of congruence in terms of a percentage value, then the required pre-determined minimum degree of congruence may be set to a level of congruence of at least 50%, e.g. at least 60%, at least 70%, or at least 80%; specifically of at least 75%, more specifically of at least 90%; and even more specifically of at least 95%. In this case, a determination of the analyte concentration in step e1) can only be carried out if the item of information from step d) indicates a percentage value for the degree of congruence equal to or above the percentage value selected as the pre-determined minimum degree of congruence (i.e. a percentage value equal to or above the applicable threshold). For example, the determination of a degree of congruence in terms of a percentage value, i.e. the extent to which the lidar measurement data set and the pre-generated lidar data set (at least partially) overlap, may be effected based on object detection within the lidar data sets. Said object detection may be evaluated by taking into account one or more parameters, e.g. by an artificial neural network, wherein the parameters may comprise accuracy, F1-score, etc. Suitable artificial neural networks may be provided e.g. by an application programming interface (API) of the mobile device, such as provided in mobile devices of Apple^{®}, e.g. in the Apple^{®} iPad Pro 2020^{®}, and in smartphones like the Apple^{®} iPhone 12 Pro^{®} and Pro Max^{®}.

The method may further comprise, after step a) and before step b1), a step b2'), corresponding to step b2); and a step c'), corresponding to step c); and a step d'), corresponding to step d); wherein, if the item of information from step d') indicates a degree of congruence above a pre-determined minimum degree of congruence, the method further comprises capturing, by the at least one camera, an initial image of the reagent test region, or of at least a part thereof, without having the sample of the bodily fluid applied thereto.

The initial image provides for a blank measurement of the test element or of the test field, respectively. This course of action allows to perform an initial check for suitability or for integrity of the test element, and specifically of the test field, prior to sample application to the test field. For example, test elements, and in particular test fields, which are deteriorated, e.g. due to an impact from environmental factors such as temperature or humidity, or because their expiry date is in the past, may be detected.

Additionally or alternatively, a blank measurement may particularly be used as a reference for the determination of the analyte concentration, and/or to perform a relative measurement for the determination of the analyte concentration. Accordingly, the method may further comprise, in step e1), taking the initial image into account for the determining of the concentration of the analyte in the bodily fluid.

The method generally takes into account that chemical reactions used for analyte detection based on color formation may require a certain amount of time to be complete, or at least to have sufficiently advanced towards completion of the chemial reaction. In other words, for the color to develop a final intensity, or at least a sufficient intensity, to be properly detected, it may be required to allow enough time for the chemical reaction to take place, or to at least sufficiently advance towards completion of the reaction. Accordingly, the method may further comprise, after step b1) and before steps e1) or e2), specifically between steps b1) and b2), a step of waiting for a predetermined minimum amount of time, such as a period of time of 5 sec or more, and up to a few minutes, e.g. 5, 10, 15, 20, 25, 30, 45, 60, 90 or 120 sec, specifically a period of time of 5 to 30 sec, and more specifically of 20 sec.

Since the lidar sensor generally is adapted to detect and to generate digital 3-D representations of a surrounding or of objects within its field of view, the processor may distinguish foreground elements from background elements in the lidar measurement data set. Specifically, such a distinction may be achieved by evaluating time-of-flight values, i.e. the times it takes for the emitted laser pulses to impinge upon an object and return to the lidar sensor, or to a receiver thereof. For example, an object which is farther away than the object to be detected (such as the at least one object) may provide for longer time-of-flight values; if said time-of-flight values exceed a specific threshold value, then the corresponding object may be classified as a background object. Thereby, any objects clearly identified as background objects may be removed from the lidar data set, or may not be taken into account for subsequent processing of the lidar data set. Additionally or alternatively, a distinction of foreground elements from background elements may be achieved by clustering one or more pluralities of time-of-flight values, wherein each of the pluralities of time-of-flight values may provide similar time-of-flight values, particularly time-of-flight values which are comprised within a specified range of time-of-flight values. More details regarding object detection and distinction of foreground elements from background elements in a lidar data set, e.g. in a lidar measurement data set, may be found in "Recognition and Tracking of Dynamic Objects Using Static Sensors", Master's Thesis by R. Zille, 2019, Human-centered Assistive Robotics, Technical University Munich.

Accordingly, the method may further comprise, in step c), identifying, by the processor, in the lidar measurement data set at least two segments. At least one of the segments is identified as a segment containing foreground elements, and at least another one of the segments is identified as a segment containing background elements, relative to one another. Thereby, the foreground elements are identified as being closer in distance to the mobile device as compared to the background elements, the background elements thus being located farther away from the mobile device than the foreground elements.

Any objects in the field of view of the lidar sensor may be represented in the lidar measurement data set; accordingly, each of said objects may be identified as either a foreground element or as a background element, and may be assigned to a corresponding segment within the lidar measurement data set. Thereby, two groups of segments may result, wherein the first group contains all segments having foreground elements, and the second group contains all segments having background elements. If appropriate, additional segments may be defined, e.g. containing elements which are located at an intermediate distance from the mobile device, i.e. at a distance farther away from the mobile device than the foreground elements, but closer to the mobile device than the background elements. Of course, this additional distinction may depend on the relevant capabilities of any specific lidar sensor or lidar system, such as its resolution capacity.

Specifically, the method may comprise detecting, by the processor, the at least one object, in the lidar measurement data set from step c), in a segment containing background elements; and the method may further comprise detecting, by the processor, at least a partial overlap of a foreground element with at least a part of the object. Such a foreground element, e.g. a user's finger, represents an obstacle which obstructs detection of the complete object by the lidar sensor. In such a case, the lidar measurement data set represents a three-dimensional structure of only a part of the object, namely the part of the object which is within the field of view of the lidar sensor and which is not obstructed by a foreground element.

It may be advantageous, particularly in order to improve the efficiency, reliability and/or performance speed of the method according to the present invention, to use, in addition to the data from the lidar sensor, particularly in addition to the lidar measurement data, image data from the at least one camera. To this end, it is beneficial if the additional image data corresponds to the lidar measurement data, particularly with respect to the point in time when each of said data is received or recorded.

Therefore, in step d), for the comparing of the lidar measurement data set from step c) to the pre-generated lidar data set for the object, the method may further comprise superimposing, by the processor, the lidar measurement data set from step c) with corresponding image data received from the at least one camera. Thereby a combined measurement data set is obtained. The combined measurement data set may be used as the lidar measurement data set for the comparing in step d). It is noted that, in this context, the term "the lidar measurement data set" accordingly explicitly refers to data received both from the lidar sensor and from the camera, respectively.

Specifically, for the superimposing by the processor in step d), it is particularly advantageous if the method further comprises selecting the lidar measurement data set from step c) and the corresponding image data received from the at least one camera, such that data relating to essentially the same point in time is superimposed.

In case the lidar measurement data set from step c) is superimposed in step d) with corresponding image data from the camera, it is advantageous for the comparing in step d), if the pre-generated lidar data set also comprises corresponding image data received from a camera, specifically from the at least one camera. In other words, for generating the pre-generated lidar data set, data received from a lidar sensor, specifically from the at least one lidar sensor of the mobile device, may be superimposed with corresponding image data received from a camera, specifically from the at least one camera of the mobile device, thereby obtaining a combined pre-generated lidar data set. The combined pre-generated lidar data set can then be used as the pre-generated lidar data set for the comparing in step d).

As the skilled person will appreciate, the method may further comprise, particularly in step c) and/or in step d), applying, by the processor, one or more image processing techniques to the lidar measurement data set, to the image data received from the at least one camera, to the combined measurement data set, and/or to a part of any of the foregoing. The term "image processing techniques" includes any conventional technique known in the field, such as color inversion; luma inversion; adjustment of contrast, brightness, etc.; thresholding; and the like. Such image processing techniques may, at least partially, be applied to data received from a lidar sensor, either directly, analogously, or in a modified manner.

In another aspect of the present invention, a mobile device having at least one camera, at least one lidar sensor, at least one processor, and at least one display is provided. The at least one camera and the at least one lidar sensor comprise an at least partially overlapping, or an essentially fully overlapping, field of view. The mobile device is configured for determining a concentration of an analyte in a bodily fluid by capturing at least one image of at least a part of (i) an optical test element having a reagent test region, (ii) a color reference card associated with such an optical test element having a reagent test region, or (iii) a color reference card having a reagent test region, by using the camera. Furthermore, the mobile device is configured for determining the concentration of the analyte in the bodily fluid by determining the at least one analyte concentration from a color formation reaction at the reagent test region. The mobile device further is configured for performing at least steps b1), b2), c), d), e1) and e2) of the analytical method as described herein above.

In another aspect of the present invention, a kit is provided, comprising a mobile device as described herein above, and at least one object being selected from the list comprising: an optical test element having a reagent test region, a color reference card having a reagent test region, a color reference card adapted to be associated with an optical test element having a reagent test region. The reagent test region is adapted for application of a sample of a bodily fluid. The reagent test region is further adapted to undergo, at least partially, a color formation reaction when the sample of the bodily fluid is applied to the reagent test region.

In another aspect of the present invention, a computer program is provided, comprising instructions which, when the program is executed by the mobile device as described herein above, cause the mobile device to carry out at least steps b1), b2), c), d), e1) and e2) of the analytical method described herein above.

In another aspect of the present invention, a computer-readable storage medium is provided, comprising instructions which, when executed by the mobile device as described herein above, cause the mobile device to carry out at least steps b1), b2), c), d), e1) and e2) of the analytical method described herein above.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a schematic view of a mobile device, and of a color reference card which is associated with an optical test strip, in a perspective view; and
- Figure 2: shows a flow chart of an embodiment of an analytical method for determining a concentration of an analyte in a bodily fluid.

### Detailed description of the embodiments

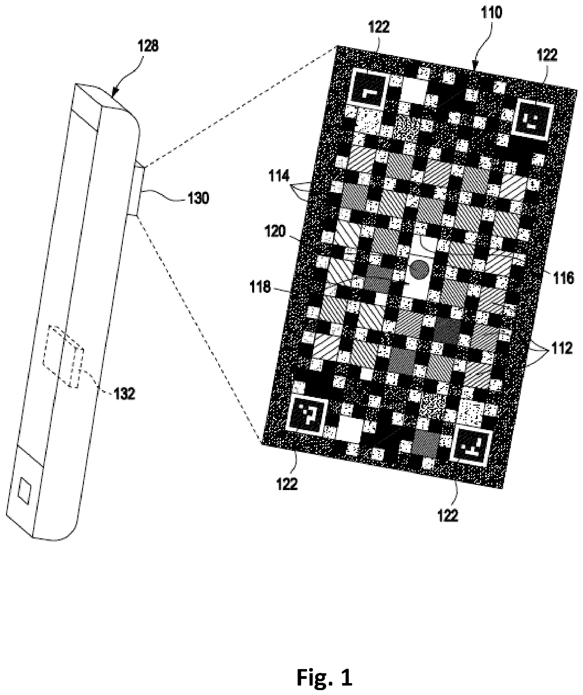

Fig. 1 shows a schematic view of a mobile device (128), and of a color reference card (110) which is associated with an optical test strip (118), in a perspective view. The optical test strip (118) is associated with the color reference card (110) such that a reagent test field (120) of the optical test strip (118) is aligned with a cut-out portion (116) of the color reference card (110). Thereby, the reagent test field (120) is visible from the top through the cut-out portion (116). The optical test strip (118) may be detachably connected to the bottom surface of the color reference card (110), e.g. by some fixation element on the bottom surface which keeps the optical test strip (118) in a fixed position relative to the color reference card (110). Alternatively, the optical test strip (118) may simply be placed below the color reference card (110) such that the reagent test field (120) is aligned with the cut-out portion (116), and such that the reagent test field (120) is visible from the top through the cut-out portion (116).

The mobile device (128) is equipped with a camera (130a) and a lidar sensor (130b), both of which are depicted in Fig. 1 in sensor unit (130). Additional cameras and/or lidar sensors may be provided in the mobile device. The mobile device (128) may be, or may comprise, at least one of a cell phone, a smartphone, a tablet computer or the like. The camera (130a) of the mobile device (128) may be configured for recording images (herein also referred to as "capturing images"), specifically color images. Thus, the camera (130a) may be a color camera, and may comprise at least three color sensors, such as at least one color sensor for the R, G, B colors. The lidar sensor (130a) of the mobile device (128) may be configured for providing output data to the processor (132), which output data allows the processor (132) to generate a lidar data set, such as a lidar measurement data set or a pre-generated lidar data set, representing a three-dimensional structure of at least one object, or of a part thereof, in the field of view of the lidar sensor (130b). Additionally or alternatively, the lidar sensor (130b) may be configured for providing output data to the processor (132), wherein the output data already is fully or partially in the form of a lidar data set, such as a lidar measurement data set or a pre-generated lidar data set, representing a three-dimensional structure of at least one object, or of a part thereof. Further, the mobile device (128) has a display (not depicted in Fig. 1) on a side of the mobile device (128) opposite to the side where the sensor unit (130) is located.

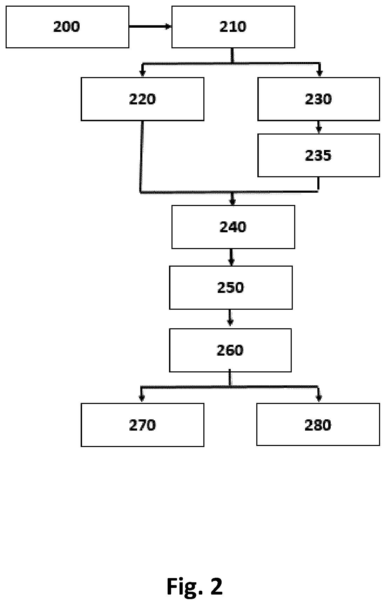

Further, the mobile device (128) generally comprises at least one processor (132). The processor (132) may be configured, specifically by software programming, to perform one or more of the method steps of the method for determining the concentration of an analyte in a bodily fluid according to the invention. An exemplary embodiment of said method is shown in Figure 2, and will be described in further detail below. The processor (132) may specifically be configured for supporting the capturing of at least one image of the color reference card (110). Specifically, the processor (132) may prompt a user of the mobile device (128) to capture one or more images. Additionally or alternatively, the processor (132) may be configured for automatically capturing an image of the color reference card (110), specifically when the color reference card (110) is in the field of view of the camera (130a).

The top surface of the color reference card (110) comprises a plurality of reference fields (color reference fields 112 and gray reference fields 114) having known reference color values or known reference gray values. Further, the color reference card (110) as depicted in Fig. 1 comprises a cut-out portion (116). Thus, the optical test strip (118) may be visible through the cut-out portion (116), specifically if the optical test strip (118) is connected to the color reference card (110), such that both, the top surface of the color reference card (110) and the optical test strip (118), may be visible on a measurement image captured by the camera (130) of the mobile device (128). Specifically, the at least one reagent test region (120) of the optical test strip (118) may be visible through the cut-out portion (116), if looking from the direction of the top surface of the color reference card (110). In such an arrangement, the sample of bodily fluid is to be applied to the reagent test region (120) of the optical test strip (118) from the direction of the bottom surface. The color formed in the reagent test region (120) is then visible through the cut-out portion (116). Additionally or alternatively, the sample of bodily fluid may be applied to the optical test strip before it is connected to the color reference card (110). Furthermore, additionally or alternatively, an optical test strip having a capillary for receiving the sample of bodily fluid and/or for transporting the sample of bodily fluid to the reagent test region (120) may be used together with the color reference card (110).

The top surface of the color reference card (110) may further comprise one or more position detection code elements (122). The position detection code elements (122) may be used for identifying the orientation of the color reference card (110) and of the top surface thereof, relative to the camera of the mobile device. Specifically, the processor (132) of the mobile device (128) may be configured for detecting the position detection code elements (122) on an image captured by the camera (130), e.g. on a measurement image, and for further retrieving information about the orientation of the color reference card (110) and of the top surface thereof.

In Fig. 2, a flow chart of an exemplary embodiment of a method for determining a concentration of an analyte, such as blood glucose, in a bodily fluid, such as blood, is shown. The method comprises using at least one mobile device (128) having at least one camera (130a) and at least one lidar sensor (130b), at least one processor (132), and at least one display (not depicted), wherein the at least one camera (130a) and the at least one lidar sensor (130b) comprise an at least partially overlapping field of view. The method further comprises using at least one object, such as a color reference card (110) associated with an optical test strip (118), as described herein above.

The method comprises the following steps, which specifically may be performed in the given order. Still, a different order may also be possible. It may be possible to perform two or more of the method steps fully or partially simultaneously. It may further be possible to perform one, more than one or even all of the method steps once or repeatedly. The method may comprise additional method steps that are not listed.

The method comprises the following steps:
a) (denoted with reference number 200) providing at least one object, for example a color reference card (110) which is associated with an optical test element (118) having a reagent test region (120);
b1) (denoted with reference number 200 as well) prompting, by the display, a user to apply a drop of the bodily fluid to the reagent test region (120);
b2) (denoted with reference number 210) prompting, by the display, the user to provide the at least one object (110, 118) within the at least partially overlapping field of view of the at least one camera (130a) and the at least one lidar sensor (130b);
c) (denoted with reference numbers 230, 235) generating, by the processor, a lidar measurement data set at least for the object (110, 118) by receiving output data from the at least one lidar sensor (130b), the lidar measurement data set representing a three-dimensional structure of at least a part of the object (110, 118);
d) (denoted with reference number 260) comparing, by the processor, the lidar measurement data set from step c) to a pre-generated lidar data set for the object (110, 118), the pre-generated lidar data set representing a three-dimensional structure of the entire object (110, 118), thereby obtaining an item of information on a degree of congruence of the lidar measurement data set and the pre-generated lidar data set; and
e1) (denoted with reference number 270) if the item of information from step d) indicates a degree of congruence equal to or above a pre-determined minimum degree of congruence: capturing, by the at least one camera (130a), a measurement image of at least a part of the reagent test region (120) having the sample of the bodily fluid applied thereto, and determining the concentration of the analyte in the bodily fluid based at least on the measurement image captured; or
e2) (denoted with reference number 280) if the item of information from step d) indicates a degree of congruence below a pre-determined minimum degree of congruence:
   - at least temporarily not allowing the capturing, by the at least one camera (130a), of a measurement image of at least a part of the reagent test region (120) having the sample of the bodily fluid applied thereto; and/or
   - indicating, by the display, a warning to the user; and/or
   - indicating, by the display, instructions to the user to take some appropriate action in order to capture, by the at least one camera (130a), a measurement image of at least a part of the reagent test region having the sample of the bodily fluid applied thereto.

In step a) (reference number 200), the at least one object may alternatively be selected to be one of: an optical test element having a reagent test region, or a color reference card having a reagent test region. The reagent test region (120) is adapted for application of a sample of the bodily fluid. Further, the reagent test region (120) is adapted to undergo, fully or at least partially, a color formation reaction when the sample of the bodily fluid is applied to the reagent test region.

Additionally or alternatively, step b1) (reference number 200), may comprise prompting, by the display, a user to confirm application of a drop of the bodily fluid to the reagent test region.

In step b2) (denoted with reference number 210), when the user has provided the at least one object (110, 118) within the at least partially overlapping field of view, the at least one lidar sensor (130b) can acquire lidar input data for one or more objects, or at least of a part thereof, within its field of view. Specifically, the lidar sensor (130b) may acquire lidar input data for the at least one object (110, 118), or of at least a part thereof.

In step c), firstly (reference number 230), the processor (132) receives output data from the at least one lidar sensor (130b). The output data may contain information on a three-dimensional structure of one or more objects, or at least of a part thereof, within the field of view of the lidar sensor (130b). Specifically, the output data may contain information on a three-dimensional structure of the at least one object (110, 118), or of at least a part thereof. Next (reference number 235), the processor (132) may generate, from the output data received from the at least one lidar sensor (130b), a lidar measurement data set at least for the object (110, 118), wherein the lidar measurement data set represents a three-dimensional structure of at least a part of the object (110, 118). Additionally, step c) (reference number 235), may comprise identifying, by the processor (132), in the lidar measurement data set at least two segments, wherein at least one of the segments is identified as a segment containing foreground elements, and wherein at least another one of the segments is identified as a segment containing background elements, relative to one another. Thereby, the processor may determine, in the lidar measurement data set, background elements and foreground elements, and may distinguish them from one another.

In step d), for the comparing of the lidar measurement data set from step c) to a pre-generated lidar data set for the object (reference number 260), the method may additionally comprise: superimposing, by the processor (132), the lidar measurement data set from step c) with corresponding image data received from the at least one camera (130a). Thereby, a combined measurement data set may be obtained. The combined measurement data set may then be used as the lidar measurement data set for the comparing in step d) (reference number 260).

To this end, image data, specifically corresponding image data, is received by the at least one camera (130a), (reference numeral 220), in addition to the lidar data received by the at least one lidar sensor (130b), (reference numeral 230). In this regard, it may be noted that the acquisition of the image data received by the camera (130a) on the one hand, and the acquisition of the lidar data received by the lidar sensor (130b) on the other hand, may be performed simultaneously, intermittently, or successively. However, it may be beneficial if the image data and the lidar data have, at least partially, essentially identical, or at least overlapping, time stamps, such that the processor (132) may determine which image data corresponds to which lidar data.

Accordingly, the lidar measurement data set from step c) may be superimposed, by the processor (132), with corresponding image data received from the at least one camera (130a). Thereby, a combined measurement data set may be obtained (reference number 240). In order to enhance or facilitate object detection and/or user handling, it may be appropriate to perform one or more image processing methods on the combined measurement data set (reference number 250), e.g. one or more of: color inversion; luma inversion; adjustment of contrast, brightness, etc.; thresholding; and the like.

The method may then proceed to step d) (reference number 260) as before. Depending on wether or not the item of information from step d) indicates a degree of congruence equal to or above the pre-determined minimum degree of congruence, the processor (132) will either allow a measurement image to be captured according to step e1) (reference number 270), or the processor (132) will initiate at least one of the options provided for at step e2) instead (reference number 280). For example, the pre-determined minimum degree of congruence may be set as high. In order to proceed to step e1), the item of information from step d) thus needs to represent a high degree of congruence (of the lidar measurement data set and the pre-generated lidar data set, each of which may additionally contain image data from the at least one camera (132)). Particularly, it may be appropriate to set the pre-determined minimum degree of congruence as high (as it is the case for the purpose of the present example), if the item of information from step d) is defined to be selected from a group consisting of two elements only, namely of a low degree of congruence and of a high degree of congruence, respectively.

List of reference numbers
- 110: color reference card
- 112: color reference field
- 114: gray reference field
- 116: cut-out portion
- 118: optical test strip
- 120: reagent test region
- 122: position detection code element
- 128: mobile device
- 130: sensor unit, containing camera 130a and lidar sensor 130b
- 132: processor

## Claims

1. An analytical method for determining a concentration of an analyte in a bodily fluid by using a mobile device having at least one camera, at least one lidar sensor, at least one processor, and at least one display, wherein the at least one camera and the at least one lidar sensor comprise an at least partially overlapping field of view, the method comprising the following steps:
a) providing at least one object, the at least one object being selected from the list comprising: an optical test element having a reagent test region, a color reference card having a reagent test region, a color reference card adapted to be associated with an optical test element having a reagent test region; wherein the reagent test region is adapted for application of a sample of the bodily fluid, and wherein the reagent test region is adapted to undergo, at least partially, a color formation reaction when the sample of the bodily fluid is applied to the reagent test region;
b1) prompting, by the display, a user to apply a drop of the bodily fluid to the reagent test region and/or prompting, by the display, a user to confirm application of a drop of the bodily fluid to the reagent test region;
b2) prompting, by the display, the user to provide the at least one object within the at least partially overlapping field of view of the at least one camera and the at least one lidar sensor;
c) generating, by the processor, a lidar measurement data set at least for the object by receiving output data from the at least one lidar sensor, the lidar measurement data set representing a three-dimensional structure of at least a part of the object;
d) comparing, by the processor, the lidar measurement data set from step c) to a pre-generated lidar data set for the object, the pre-generated lidar data set representing a three-dimensional structure of the entire object, thereby obtaining an item of information on a degree of congruence of the lidar measurement data set and the pre-generated lidar data set; and
e1) if the item of information from step d) indicates a degree of congruence equal to or above a pre-determined minimum degree of congruence: capturing, by the at least one camera, a measurement image of at least a part of the reagent test region having the sample of the bodily fluid applied thereto, and determining the concentration of the analyte in the bodily fluid based at least on the measurement image captured; or
e2) if the item of information from step d) indicates a degree of congruence below a pre-determined minimum degree of congruence:
- at least temporarily not allowing the capturing, by the at least one camera, of a measurement image of at least a part of the reagent test region having the sample of the bodily fluid applied thereto; and/or
- indicating, by the display, a warning to the user; and/or
- indicating, by the display, instructions to the user to take some appropriate action in order to capture, by the at least one camera, a measurement image of at least a part of the reagent test region having the sample of the bodily fluid applied thereto.

2. The method according to claim 1, further comprising, after step a) and before step b1), a step b2'), corresponding to step b2); and a step c'), corresponding to step c); and a step d'), corresponding to step d); wherein, if the item of information from step d') indicates a degree of congruence above a pre-determined minimum degree of congruence, the method further comprises capturing, by the at least one camera, an initial image of at least a part of the reagent test region without having the sample of the bodily fluid applied thereto.

3. The method according to claim 2, further comprising, in step e1), taking the initial image into account for the determining of the concentration of the analyte in the bodily fluid.

4. The method according to any one of claims 2 and 3, further comprising, after step b1) and before steps e1) or e2), a step of waiting for a predetermined minimum amount of time.

5. The method according to any one of the preceding claims, further comprising, in step c), identifying, by the processor, in the lidar measurement data set at least two segments, wherein at least one of the segments is identified as a segment containing foreground elements, and wherein at least another one of the segments is identified as a segment containing background elements, relative to one another.

6. The method according to any one of the preceding claims, further comprising, in step d), for the comparing of the lidar measurement data set from step c) to a pre-generated lidar data set for the object: superimposing, by the processor, the lidar measurement data set from step c) with corresponding image data received from the at least one camera, thereby obtaining a combined measurement data set; and using the combined measurement data set as the lidar measurement data set for the comparing in step d).

7. The method according to claim 6, further comprising, for the superimposing by the processor, selecting the lidar measurement data set from step c) and the corresponding image data received from the at least one camera, such that data relating to essentially the same point in time is superimposed.

8. The method according to any one of claims 5 to 7, further comprising, in step c) and/or in step d), applying, by the processor, one or more image processing techniques to the lidar measurement data set, to the image data received from the at least one camera, to the combined measurement data set, and/or to a part of any of the foregoing.

9. The method according to any one of claims 5 to 8, further comprising: detecting, by the processor, the object, in the lidar measurement data set from step c), in a segment containing background elements; and detecting, by the processor, at least a partial overlap of a foreground element with at least a part of the object.

10. The method according to any one of the preceding claims, wherein, if the item of information from step d) is selected from a group consisting of a low degree of congruence and a high degree of congruence, the pre-determined minimum degree of congruence is set as high; or wherein, if the item of information from step d) is selected from a group consisting of a low degree of congruence, a medium degree of congruence, and a high degree of congruence, the pre-determined minimum degree of congruence is set as medium or as high; or wherein, if the item of information from step d) is selected to represent the degree of congruence in terms of a percentage value, then the required pre-determined minimum degree of congruence is set to a level of congruence of at least 50%.

11. The method according to any one of the preceding claims, wherein, in step e2), the appropriate action to be taken by the user is at least one of: clearing the overlapping field of view, at least in between the camera and the at least one object, of any obstacles; moving the mobile device in at least one direction, relative to the at least one object; and changing an angle of orientation of the mobile device relative to the at least one object; such that the overlapping field of view, at least in between the camera and the at least one object, is clear of any obstacles.

12. A mobile device having at least one camera, at least one lidar sensor, at least one processor, and at least one display, wherein the at least one camera and the at least one lidar sensor comprise an at least partially overlapping field of view, the mobile device being configured for determining a concentration of an analyte in a bodily fluid by capturing at least one image of at least a part of (i) an optical test element having a reagent test region, (ii) a color reference card associated with such an optical test element having a reagent test region, or (iii) a color reference card having a reagent test region, by using the camera, and by determining the at least one analyte concentration from a color formation reaction at the reagent test region, wherein the mobile device further is configured for performing at least steps b1), b2), c), d), e1) and e2) of the analytical method according to any one of claims 1 to 11.

13. A kit, comprising a mobile device according to claim 12, and at least one object being selected from the list comprising: an optical test element having a reagent test region, a color reference card having a reagent test region, a color reference card adapted to be associated with an optical test element having a reagent test region; wherein the reagent test region is adapted for application of a sample of a bodily fluid, and wherein the reagent test region is adapted to undergo, at least partially, a color formation reaction when the sample of the bodily fluid is applied to the reagent test region.

14. A computer program comprising instructions which, when the program is executed by the mobile device according to claim 12, cause the mobile device to carry out at least steps b1), b2), c), d), e1) and e2) of the analytical method of any one of claims 1 to 11.

15. A computer-readable storage medium comprising instructions which, when executed by the mobile device according to claim 12, cause the mobile device to carry out at least steps b1), b2), c), d), e1) and e2) of the analytical method of any one of claims 1 to 11.
